# EUROPEAN PATENT SPECIFICATION

(11) **EP 2 452 673 B1**
(45) Date of publication and mention of the grant of the patent: **26.04.2017**
(21) Application number: 09702300.6
(22) Date of filing: 21.01.2009
(51) Int. Cl.: A61K 9/06, A61K 36/324, A61K 36/28, A61K 31/722, A61K 36/36, A61K 36/185, A61P 17/02, A61K 36/328

(54) **CHITOSAN GEL FOR DERMATOLOGICAL USE, PRODUCTION METHOD THEREFOR AND USE OF SAME**
CHITOSAN-GEL ZUR DERMATOLOGISCHEN ANWENDUNG, HERSTELLUNGSVERFAHREN DAFÜR UND SEINE VERWENDUNG
GEL DE CHITOSANE POUR APPLICATIONS DERMATOLOGIQUES, PROCÉDÉ D'OBTENTION ET D'UTILISATION DE CELUI-CI

(43) Date of publication of application: 16.05.2012
(73) Proprietor: Laboratorios Synthesis S.A.S., Bogota D.C. (CO); Igloo Zone Chile S.a., Santiago de Chile (CL)
(72) Inventor: SHAUL HASSON NISIS, Ariel, Santiago de Chile (CL)
(74) Representative: Hart, Deborah Mary
(86) International application number: PCT/IB2009/050228
(87) International publication number: WO 2009/090624

(56) References cited:
- EP-A2- 0 460 385
- WO-A1-92/09636
- WO-A2-01/00246
- CN-A- 101 057 953
- JP-A- 2006 045 181
- KR-A- 20010 016 482
- KR-A- 20060 124 927
- US-A- 4 659 700
- US-A1- 2001 024 655
- US-A1- 2005 042 265
- US-A1- 2005 113 773
- US-A1- 2007 048 358
- CEREN ALEMDAROGLU ET AL: "An investigation on burn wound healing in rats with chitosan gel formulation containing epidermal growth factor", BURNS, vol. 32, no. 3, 1 May 2006 (2006-05-01), pages 319-327, XP055048264, ISSN: 0305-4179, DOI: 10.1016/j.burns.2005.10.015
- RASHED A N ET AL: "Simple evaluation of the wound healing activity of a crude extract of Portulaca oleracea L.(growing in Jordan) in Mus musculus JVI-1", MEDICINAL & AROMATIC PLANTS ABSTRACTS, SCIENTIFIC PUBLISHERS, NEW DELHI - INDIA, vol. 26, no. 6, 1 December 2004 (2004-12-01), XP018001528,
- Gerit D. Mulder, DPM, MS; Joseph P. Cavorsi, MD; Daniel K. Lee, DPM: "Polyhexamethylene Biguanide (PHMB): An Addendum to Current Topical Antimicrobials", wounds , vol. 19, no. 7 30 June 2007 (2007-06-30), XP002689622, Retrieved from the Internet: URL:http://www.woundsresearch.com/print/22 1 [retrieved on 2012-12-18]
- BOUCARD, N. ET AL.: 'The use of physical hydrogels of chitosan for skin regeneration following third-degree bums' BIOMATERIALS vol. 28, no. 24, 2007, pages 3478 - 3488, XP025321204

## Description

### DESCRIPTION OF INVENTION

Present invention relates to a chitosan-base gel and more particularly to gels formulated with excipients which allow a protective film over skin after application.

This invention relates to a gel for topical application, characterized by incorporating chitosan as the main component.

Chitosan is from natural origin, therefore it is biocompatible. It further shows antimicrobial properties (fungicide and bactericide) inherent to its intrinsec features. It corresponds to a N-acetyl-D-glucosamine deacetylation linear polysaccharide commercially obtained by chitin deacetylation.

Chitosan has been useful in heavy metal absorption in water and industrial waste flows and also in developing cosmetic preparations. Furthermore, partially acetylated chitosan derivatives have been subject of significant research in search of therapeutic substances or implantable materials. Biocompatibility of materials comprising chitosan has been specifically assessed in blood, wounds and bones.

Chitosan has been also studied in drug delivery systems through gels. (Cohya and Cois (1993) J. Microencapsulation, 10(1):1-9).

The present application relates to stable hydrophilic based polymer gels as defined in the claims.

In order to carry out the present invention a number of mixtures were used to obtain solutions that allowed gel preparation in percentages between 2% and 8% from chitosan, combined with other components conferring suitable therapeutic characteristics to product, to be applied on irritated skins, surface burns, dermal abrasions, post-peeling, post-laser and generally over eroded skins.

Chitosan (poly-β-1,4-D-glucosamine) is a natural polysaccharide derivative, known as chitin. (poly-β-1,4-D-acetylglucosamine) which is mainly found in crustacean shell exoskeleton.

Biodegradable polymer properties may be modified and improved through addition of a number of substances (chitosan, portulaca, alpha hydroxyacid pantenol and innocuous preservatives). By using components having well known biodegradability and therapeutic properties in burns and eroded and damaged skins in general, the gels supported by the invention are entirely biodegradable and with improved properties, and can be obtained at low costs.

The chitosan gels are obtained by dissolution of chitosan in organic acid solutions, mainly of the type alpha-hydroxy acids, such as: glycolic acid, lactic acid, alpha hydroxyethanoic acid, alpha hydroxyoctanoic acid, alpha hydroxycaprilic acid, malic acid, citric acid and tartaric acid, having good solubility in water. The hydrogels are obtained by using chitosan having different molecular weights between 25,000 and 1,250,000 g/mol and with a deacetylation degree higher than 80%.

The properties of the hydrogel are improved by the addition of panthenol and portulaca or another natural substance having similar characteristics, each one in a proportion varying between 0.1 and 45%.

Surprisingly, a stable preparation at a pH between 5.0 and 5.9 has been prepared with chitosan as the only one viscosifier in a percentage between 0.2 and 10%, with the addition of at least two preservatives, also including portulaca and panthenol or another natural component, which confer relevant features for the treatment of burns type or erosion type lesions of the skin, thus achieving a rapid regeneration and cicatrization of the damaged tissues.

In the sequence of pictures appearing below, the effect of the hydrogel on the damaged skin is appreciated.

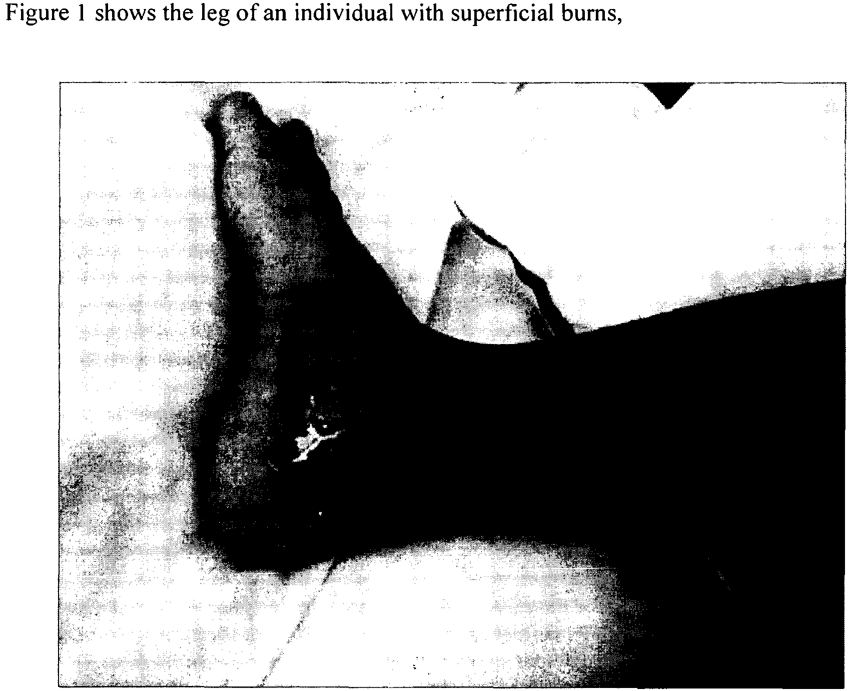

Figures 2 and 3 show the area of the leg alter the application of the hydrogel, with a coating of the protective and tissue regenerator product.

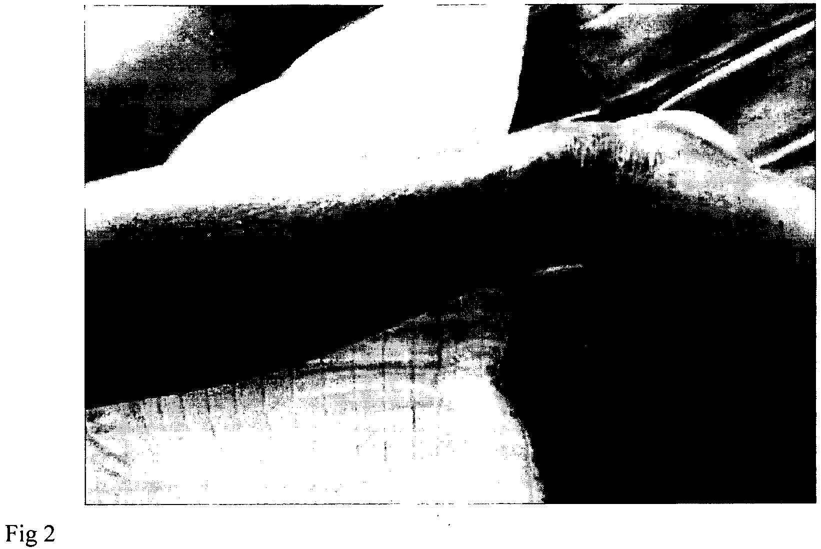

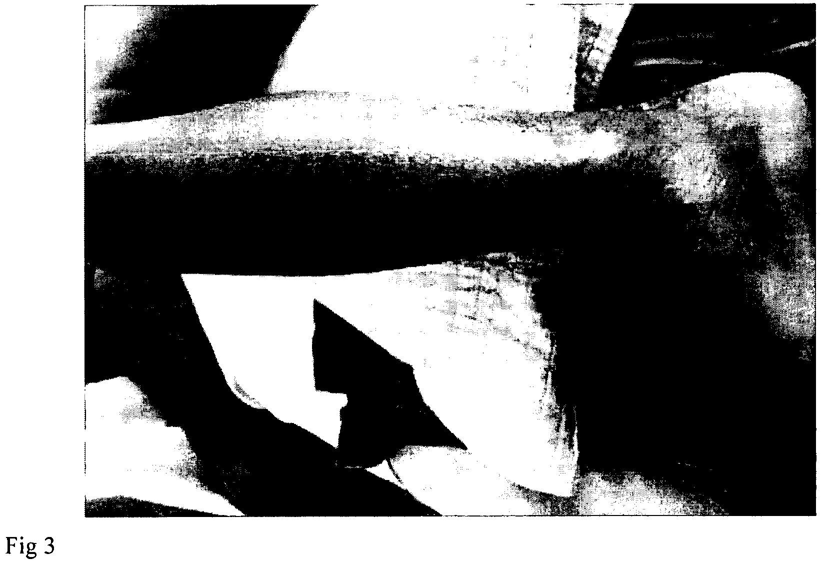

Figure 4 shows the leg area at the third day after the application of the product, with a clear recovering of the damaged tissue being observed, thus permitting to confirm the therapeutic benefits of the hydrogel.

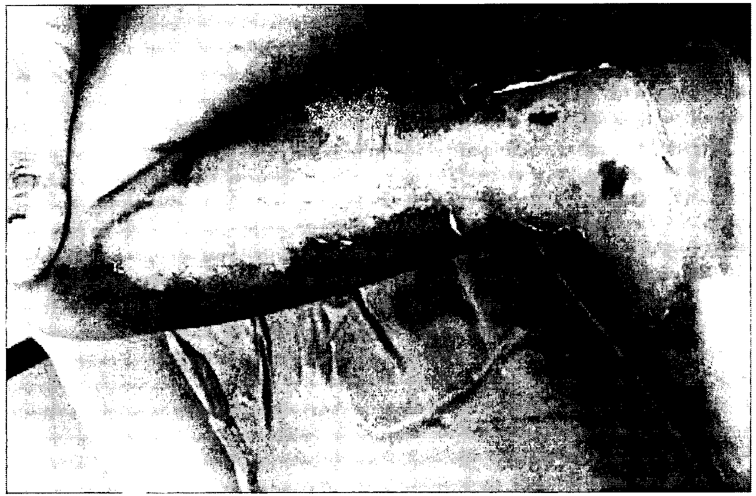

### Methodology of preparation of the hydrogel

In a stainless steel container, having distilled water, the diluted organic acid is added, it is homogenized and the pH is kept between 4.0 and 4.5, during the manufacturation of the hydrogel; after that all the chitosan, as appearing in the formulation, is slowly added under continuous stirring up to complete the dissolution.

Subsequently, the preservatives polyaminopropyl biguanide, Gluconolactone, Sodium Benzoate and Calcium Gluconate or mixtures of the other preservatives which are included in this patent, are added; subsequently the portulaca or another natural extract is added and, after that the panthenol is added.

Finally the pH is adjusted between 5.0 and 5.9 by employing an organic acid solution diluted between 1% and 2% or a 2% sodium hydroxide solution.

Subsequently, the preparation is stored in a container until fractioning.

The compositions are indicated in this patent as examples and they do not restrict the scope and protection of the invention.

### Example 1

| | |
|---|---|
| Chitosan Powder | 3.50 g |
| Polyaminopropyl biguanide | 1.50 g |
| *Portulaca oleracea* Extract | 4.00 g |
| Panthenol | 4.00 g |
| Citric acid 1% w/v q.s. | pH 5.5 |
| Distilled water q.s. | 100 g |

### Example 2

| | |
|---|---|
| Chitosan Powder | 4.0 g |
| Polyaminopropyl biguanide | 1.5 g |
| *Portulaca oleracea* Extract | 6.0 g |
| Panthenol | 4.0 g |
| Citric acid 1% w/v q.s. | pH 5.5 |
| Distilled water q.s. | 100 g |

### Example 3

| | |
|---|---|
| Chitosan Powder | 4.0 g |
| Polyaminopropyl biguanide | 1.5 g |
| *Portulaca oleracea* Extract | 4.0 g |
| Panthenol | 6.0 g |
| Lactic acid 1% w/v q.s. | pH 5.5 |
| Distilled water q.s. | 100 g |

### Example 4

| | |
|---|---|
| Chitosan Powder | 8.0 g |
| Polyaminopropyl biguanide | 1.5 g |
| *Portulaca oleracea* Extract | 4.0 g |
| Panthenol | 6.0 g |
| Citric acid 1% w/v q.s. | pH 5.5 |
| Distilled water q.s. | 100 g |

### Example 5

| | |
|---|---|
| Chitosan Powder | 3.50 g |
| Polyaminopropyl biguanide | 1.50 g |
| Gluconolactone/Sodium Benzoate/Ca⁺⁺ Gluconate | 1.50 g |
| *Portulaca oleracea* Extract | 4.00 g |
| Panthenol | 4.00 g |
| Lactic acid 1% w/v q.s. | pH 5.5 |
| Distilled water q.s. | 100 g |

### Example 6

| | |
|---|---|
| Chitosan Powder | 4.0 g |
| Polyaminopropyl biguanide | 1.50 g |
| Gluconolactone/Sodium Benzoate/ Ca⁺⁺ Gluconate | 1.50 g |
| *Portulaca oleracea* Extract | 6.00 g |
| Panthenol | 4.00 g |
| Lactic acid 1% w/v q.s. | pH 5.5 |
| Distilled water q.s. | 100 g |

### Example 7

| | |
|---|---|
| Chitosan Powder | 4.0 g |
| Polyaminopropyl biguanide | 1.50 g |
| Gluconolactone/Sodium Benzoate/ Ca⁺⁺ Gluconate | 1.50 g |
| *Portulaca oleracea* Extract | 4.00 g |
| Panthenol | 6.00 g |
| Lactic acid 1% w/v q.s. | pH 5.5 |
| Distilled water q.s. | 100 g |

### Example 8

| | |
|---|---|
| Chitosan Powder | 8.0 g |
| Polyaminopropyl biguanide | 1.50 g |
| Gluconolactone/Sodium Benzoate/ Ca⁺⁺ Gluconate | 1.50 g |
| *Portulaca oleracea* Extract | 4.00 g |
| Panthenol | 6.00 g |
| Lactic acid 1% w/v q.s. | pH 5.5 |
| Distilled water q.s. | 100 g |

**Table 1**

| | **Stability of the compositions** | | | | | | | | | | |
|---|---|---|---|---|---|---|---|---|---|---|---|
| | | | | | | | | | | | |

| | **MONTH 1** | | | | **MONTH 2** | | | | **MONTH 3** | | |
|---|---|---|---|---|---|---|---|---|---|---|---|
| Examples | viscosity | pH | Microbiology | | viscosity | pH | Microbiology | | viscosity | pH | Microbiology |
| 1 | 10,000 cps | 5.5 | lower than 100 | | 100 cps | 5.4 | higher than 1000 | | 100 cps | 5.4 | higher than 1000 |
| 2 | 12,000 cps | 5.5 | lower than 100 | | 50 cps | 5.5 | higher than 1000 | | 50 cps | 5.5 | higher than 1000 |
| 3 | 12,000 cps | 5.5 | lower than 100 | | 200 cps | 5.4 | higher than 1000 | | 200 cps | 5.2 | higher than 1000 |
| 4 | 15,000 cps | 5.5 | lower than 100 | | 200 cps | 5.3 | higher than 1000 | | 200 cps | 5.3 | higher than 1000 |
| 5 | 11,000 cps | 5.5 | lower than 100 | | 11,500 cps | 5.5 | lower than 100 | | 11,900 cps | 5.5 | lower than 100 |
| 6 | 14,000 cps | 5.5 | lower than 100 | | 14,800 cps | 5.4 | lower than 100 | | 14,600 cps | 5.4 | lower than 100 |
| 7 | 15,000 cps | 5.5 | lower than 100 | | 15,300 cps | 5.4 | lower than 100 | | 15,100 cps | 5.5 | lower than 100 |
| 8 | 18,000 cps | 5.5 | lowerthan 100 | | 18,200 cps | 5.5 | lower than 100 | | 18,100 cps | 5.5 | lower than 100 |

### Specifications of the final product:

Aspect, gel having a middle viscosity, free of strange particles in suspension, of a light yellow color and traslucid. Viscosity, values between 5.000 and 20.000 cps.
pH values between 5,0 and 5,9
Microbiologic: no mesophilic aerobic germs and pathogens, ≤ 100 UFC/gram and Fungus and Yeast ≤ 100 UFC/g;

In table 1 the importance of including the hydrogels of the preservatives mixture in the compositions may be observed, in examples 1 to 4 the compositions have lower variations, but the most important is that only one preservative is included, in examples 5 to 8, the compositions have lower variations, but the most significant is the inclusion within the components of a mixture of preservatives. From the results of the table it may be appreciated that, at the second month of the study of a significant change in the viscosity, a drastic decrease is produced in the formulations 1 to 4 as well as an evident microbiological contamination, specifically by fungi. This has not occurred in the formulations of Examples 5 to 8, which include the mixture of preservatives.

This experimental text has been carried out under normal room conditions for temperature and relative humidity ( 25°± 2°C and 60 ± 5 %H).

It is not usual and it is surprising the fact that it was necessary to use a mixture of preservatives for preserving the conditions (specifications of the product), because it would have been expected that the use of one preservative would be enough to keep the stability of the product. It was not foresaw that the use of only one preservative would result in the variations observed for the viscosity.

## Claims

1. A stable hydrophilic based polymer gel for topical application for use in treating burn injuries and skin erosions, **characterized in that**:
a) It contains chitosan, having a molecular weight between 25,000 and 1,250,000 g/mol., is the main constituent of the hydrogel and it is present in proportions varying between 2 and 8%
b) The chitosan is dissolved by the addition of a solvent,
c) It contains panthenol and *Portulaca oleracea* extract.
d) It contains the preservatives polyaminopropyl Biguanide, Gluconolactone, Sodium Benzoate and Calcium Gluconate that are innocuous to the skin each one in a concentration varying between 0.1 and 30%.
e) It has a pH between 5.0 and 5.9.

2. A gel according to claim 1 , **characterized in that** the solvent is a diluted organic acid, from the alpha hydroxyl acids family.

3. A gel according to claim 2, wherein the alpha hydroxyl acid is selected from glycolic acid, lactic acid, alpha hydroxyethanoic acid, alpha hydroxyoctanoic acid, alpha hydroxycaprilic acid, malic acid, citric acid and tartaric acid.

4. A gel according to claims 1 to 3, **characterized in that** the panthenol and portulaca oleracea are present in a percentage varying between 0.1 and 45% each.

5. Method for obtaining the gel for topical use of claims 1 to 4, **characterized by** the following steps:
a) Preparing the solutions of chitosan, from a 1-2%, w/v solution of a weak organic acid containing between 2 and 8% w/w of chitosan.
b) Adding to the solution obtained in (a) the mixture of preservatives in a proportion varying between 0.1 and 30 %.
c) Then, once the mixture is homogeneous, adding under continuous stirring, the *Portulaca oleracea* extract and the panthenol in a proportion varying between 0.1 and 45% each.
d) Finally, adjusting the pH between 5.0 and 5.9 with a 2% sodium hydroxide solution or an organic acid solution diluted between 1 and 2%.

6. Method for obtaining an hydrochitosan gel, according to claim 5, **characterized in that**, as organic acid, an alpha hydroxy acid is employed.

7. The method of claim 6 wherein the alpha hydroxyl acid is selected from glycolic acid, lactic acid, alpha hydroxyethanoic acid, alpha hydroxyoctanoic acid, alpha hydroxycaprilic acid, malic acid , citric acid and tartaric acid.

## Patentansprüche

1. Stabiles hydrophil basiertes Polymergel für die topische Anwendung zur Verwendung bei der Behandlung von Brandverletzungen und Hauterosionen, **dadurch gekennzeichnet, dass**:
a) es Chitosan mit einem Molekulargewicht von zwischen 25.000 und 1.250.000 g/mol als dem Hauptbestandteil des Hydrogels enthält, welches in zwischen 2 und 8% variierenden Anteilen vorhanden ist,
b) das Chitosan durch den Zusatz eines Lösungsmittels gelöst ist,
c) es Panthenol und *Portulaca oleracea*-Extrakt enthält,
d) es die Konservierungsmittel Polyaminopropylbiguanid, Gluconolacton, Natriumbenzoat und Calciumgluconat, welche unschädlich für die Haut sind, jeweils in einer zwischen 0,1 und 30% variierenden Konzentration enthält,
e) es einen pH-Wert zwischen 5,0 und 5,9 aufweist.

2. Gel gemäß Anspruch 1, **dadurch gekennzeichnet, dass** das Lösungsmittel eine verdünnte organische Säure aus der Familie der Alphahydroxysäuren ist.

3. Gel gemäß Anspruch 2, wobei die Alphahydroxysäure ausgewählt ist aus Glykolsäure, Milchsäure, alpha-Hydroxyethansäure, alpha-Hydroxyoctansäure, alpha-Hydroxycaprylsäure, Apfelsäure, Zitronensäure und Weinsäure.

4. Gel gemäß Ansprüchen 1 bis 3, **dadurch gekennzeichnet, dass** Panthenol und *Portulaca oleracea* jeweils in einem zwischen 0,1 und 45% variierenden prozentualen Anteil vorhanden sind.

5. Verfahren zum Erhalt des Gels für die topische Anwendung gemäß Ansprüchen 1 bis 4, **gekennzeichnet durch** die folgenden Schritte:
a) Herstellen der Lösungen von Chitosan aus einer 1-2%igen (w/v) Lösung einer schwachen organischen Säure, die zwischen 2 und 8% (w/v) Chitosan enthält.
b) Zugeben zu der in (a) erhaltenen Lösung des Gemischs aus Konservierungsmitteln in einem zwischen 0,1 und 30% variierenden Anteil.
c) Dann, sobald das Gemisch homogen ist, Zugeben unter fortgesetztem Rühren des *Portulaca oleracea*-Extrakts und des Panthenols jeweils in einem zwischen 0,1 und 45% variierenden Anteil.
d) Abschließend Einstellen des pH-Werts auf zwischen 5,0 und 5,9 mit einer 2%igen Natriumhydroxidlösung oder einer auf zwischen 1 und 2% verdünnten Lösung einer organischen Säure.

6. Verfahren zum Erhalt eines Chitosanhydrogels gemäß Anspruch 5, **dadurch gekennzeichnet, dass**, als der organischen Säure, eine Alphahydroxysäure verwendet wird.

7. Verfahren gemäß Anspruch 6, wobei die Alphahydroxysäure ausgewählt wird aus Glykolsäure, Milchsäure, alpha-Hydroxyethansäure, alpha-Hydroxyoctansäure, alpha-Hydroxycaprylsäure, Apfelsäure, Zitronensäure und Weinsäure.

## Revendications

1. Gel polymère à base hydrophile stable pour application topique destiné à être utilisé dans le traitement des brûlures et des érosions cutanées, **caractérisé en ce que** :
a) Il contient du chitosane, qui a un poids moléculaire situé entre 25 000 et 1 250 000 g/mol., qui est le constituant principal de l'hydrogel et qui est présent en des proportions variant entre 2 et 8 %
b) Le chitosane est dissous par l'ajout d'un solvant,
c) Il contient du panthénol et un extrait de *Portulaca oleracea.*
d) Il contient les agents de conservation biguanide de polyaminopropyle, gluconolactone, benzoate de sodium et gluconate de calcium qui sont inoffensifs pour la peau, chacun en une concentration variant entre 0,1 et 30 %.
e) Il a un pH situé entre 5,0 et 5,9.

2. Gel selon la revendication 1, **caractérisé en ce que** le solvant est un acide organique dilué, de la famille des acides alpha hydroxyliques.

3. Gel selon la revendication 2, dans lequel l'acide alpha hydroxylique est choisi parmi l'acide glycolique, l'acide lactique, l'acide alpha hydroxyéthanoïque, l'acide alpha hydroxyoctanoïque, l'acide alpha hydroxycaprilique, l'acide malique, l'acide citrique et l'acide tartrique.

4. Gel selon les revendications 1 à 3, **caractérisé en ce que** le panthénol et portulaca oleracea sont présents en un pourcentage variant entre 0,1 et 45 % chacun.

5. Procédé permettant d'obtenir le gel à usage topique selon les revendications 1 à 4, **caractérisé par** les étapes suivantes :
a) Préparation des solutions de chitosane, à partir d'une solution à 1-2 %, en p/v, d'un acide organique faible contenant entre 2 et 8 % en p/p de chitosane.
b) Ajout à la solution obtenue en (a) du mélange d'agents de conservation en une proportion variant entre 0,1 et 30 %.
c) Puis, une fois que le mélange est homogène, ajout sous agitation continue de l'extrait de *Portulaca oleracea* et du panthénol en une proportion variant entre 0,1 et 45 % chacun.
d) Enfin, ajustement du pH entre 5,0 et 5,9 à l'aide d'une solution d'hydroxyde de sodium à 2 % ou d'une solution d'acide organique diluée entre 1 et 2 %.

6. Procédé permettant d'obtenir un gel d'hydrochitosane, selon la revendication 5, **caractérisé en ce que**, en tant qu'acide organique, un alpha hydroxy acide est employé.

7. Procédé selon la revendication 6 dans lequel l'acide alpha hydroxylique est choisi parmi l'acide glycolique, l'acide lactique, l'acide alpha hydroxyéthanoïque, l'acide alpha hydroxyoctanoïque, l'acide alpha hydroxycaprilique, l'acide malique, l'acide citrique et l'acide tartrique.
